# EUROPEAN PATENT APPLICATION

(11) **EP 3 711 754 A1**
(43) Date of publication of application: **23.09.2020**
(21) Application number: 20159407.4
(22) Date of filing: 20.10.2012
(51) Int. Cl.: A61K 31/282, A61K 36/24, A61P 35/00, A61K 31/555, A61K 31/665, A61K 33/24, A61K 36/886

(54) **THERAPEUTIC COMBINATION FOR THE TREATMENT OF CANCER COMPRISING PLATINUM COMPOUND AND EXTRACT OF NERIUM SPECIES**

(30) Priority: 20.10.2011 US 201161549386 P
(62) Divisional of application: 12841023.0
(71) Applicant: Nerium Biotechnology, Inc., San Antonio, TX 78230 (US)
(72) Inventor: KNOCKE, Dennis R., Shavano Park, TX Texas 78230 (US); NESTER, Joseph B., San Antonio, TX Texas 78249 (US); PAPASOTIRIOU, Ioannis, 53070 Filotas (GR)
(74) Representative: Mewburn Ellis LLP

(57) **Abstract**

This invention relates to therapeutic combinations comprising a platinum-based antineoplastic agent, such as cisplatin, and an extract from a species of the genus *Nerium,* such as *Nerium oleander,* as well as methods of using such combinations to treat subjects, including humans, suffering from certain cancers, such as prostate cancer, melanoma, pancreatic cancer, lung cancer, breast cancer, or colorectal cancer.

## Description

### FIELD OF THE INVENTION

This invention relates to therapeutic combinations comprising a platinum-based anti-neoplastic agent and an extract from a species of the genus *Nerium,* as well as methods of using such combinations to treat patients suffering from certain types of cancer.

### BACKGROUND OF THE INVENTION

Because of the continuing need for more effective ways to treat cancer, great effort continues to be exerted to develop new drugs that are less toxic to the patient being treated. However, another approach is to try to lower the toxicity of existing anti-cancer drugs by combining them with other drugs.

Platinum-based anti-cancer drugs, such as cisplatin, find use in chemotherapy of various types of cancer, but their toxicity remains a serious problem. Furthermore, the emergence of cisplatin resistance in some tumors vitiates its utility at the toxicity-limited dosage. To mitigate these limitations, cisplatin is commonly used in combination with other drugs, such as 5-fluorouracil, that exert their toxic effects on different organs than those affected by cisplatin.

Extracts of *Nerium oleander* comprise various polysaccharides and proteins as well as two toxic cardiac glycosides, oleandrin and oleandrigenin, that are known to exhibit anti-cancer activity.

### EMBODIMENTS OF THE INVENTION

An embodiment of the invention is a therapeutic combination comprising (I) an anti-neoplastic agent comprising platinum and (II) an extract from a species of the genus *Nerium.*

Another embodiment of the invention is the above therapeutic combination, wherein (I) is present in an amount that, in the absence of (II), is effective to produce an increase in anti-neoplastic activity.

Another embodiment of the invention is the above therapeutic combination, wherein (I) is present in an amount that, in the absence of (II), is ineffective to produce an increase in anti-neoplastic activity.

Another embodiment of the invention is the above therapeutic combination, wherein (I) is selected from the group consisting of cisplatin, carboplatin, oxaliplatin, satraplatin, picoplatin, nedaplatin, triplatin, and phosphaplatins.

Another embodiment of the invention is the above therapeutic combination, wherein (I) is cisplatin.

Another embodiment of the invention is the above therapeutic combination, wherein (II) is an extract from a species selected from the group consisting of *Nerium indicum, Nerium oleander,* and *Nerium odorum.*

Another embodiment of the invention is the above therapeutic combination, wherein (II) is an extract from the species *Nerium oleander.*

Another embodiment of the invention is the above therapeutic combination, wherein (II) is a water-based extract.

Another embodiment of the invention is the above therapeutic combination, wherein (II) is an aloe-based extract,

Another embodiment of the invention is the above therapeutic combination, wherein (I) is cisplatin and (II) is an extract from the species *Nerium oleander.*

Another embodiment of the invention is the above therapeutic combination, wherein (I) and (II) are present as separate formulations.

Yet another embodiment of the invention is a method for treating cancer comprising administering to a patient in need thereof a therapeutic combination comprising (I) an anti-neoplastic agent comprising platinum and (II) an extract from a species of the genus *Nerium.*

Another embodiment of the invention is the above method, wherein (I) is present in an amount that, in the absence of (II), is effective to produce an increase in anti-neoplastic activity.

Another embodiment of the invention is the above method, wherein (I) is present in an amount that, in the absence of (II), is ineffective to produce an increase in anti-neoplastic activity.

Another embodiment of the invention is the above method, wherein (I) is selected from the group consisting of cisplatin, carboplatin, oxaliplatin, satraplatin, picoplatin, nedaplatin, triplatin, and phosphaplatins.

Another embodiment of the invention is the above method, wherein (I) is cisplatin.

Another embodiment of the invention is the above method, wherein (II) is an extract from a species selected from the group consisting of *Nerium indicum, Nerium oleander,* and *Nerium odorum.*

Another embodiment of the invention is the above method, wherein (II) is an extract from the species *Nerium oleander.*

Another embodiment of the invention is the above method, wherein (II) is a water-based extract.

Another embodiment of the invention is the above method, wherein (II) is an aloe-based extract.

Another embodiment of the invention is the above method, wherein (I) is cisplatin and (II) is an extract from the species *Nerium oleander.*

Another embodiment of the invention is the above method, wherein (I) and (II) of said therapeutic combination are administered to the patient simultaneously, separately, or sequentially.

Another embodiment of the invention is the above method, wherein (II) is administered to the patient intramuscularly, sublingually, or intramuscularly and sublingually.

Another embodiment of the invention is the above method, wherein (II) is administered to the patient sublingually in two or more doses.

Another embodiment of the invention is the above method, wherein the cancer treated is prostate cancer, melanoma, pancreatic cancer, lung cancer, breast cancer, or colorectal cancer.

### BRIEF DESCRIPTION OF THE DRAWINGS

Figure 1 depicts a graph of the relative reduction in cells of the LNCaP prostate cancer cell line in the presence of Anvirzel™, cisplatin, and a combination of the two.
Figure 2 depicts a graph of the relative reduction in cells of the A375 melanoma cancer cell line in the presence of Anvirzel™, cisplatin, and a combination of the two.
Figure 3 depicts a graph of the relative reduction in cells of the PANC-1 pancreatic cancer cell line in the presence of Anvirzel™, cisplatin, and a combination of the two,
Figure 4 depicts a graph of the relative reduction in cells of the COLO699N lung cancer cell line in the presence of Anvirzel™, cisplatin, and a combination of the two.

### DETAILED DESCRIPTION OF THE INVENTION

It has surprisingly been found that combinations of an anti-neoplastic agent comprising platinum and an extract from a species of the genus *Nerium* synergistically exhibit increased anti-neoplastic activity. As a result, otherwise ineffective but well-tolerated doses of such platinum-based anti-neoplastic agents, when combined with dosages of the extract that lack efficacy themselves, exert clinically useful anti-neoplastic efficacy.

The therapeutic combinations of this invention comprise an anti-neoplastic agent comprising platinum and an extract from a species of the genus *Nerium.*

The anti-neoplastic agent used in the therapeutic combinations of this invention can be any known anti-cancer or anti-tumor agent or drug that contains platinum. Non-limiting examples include cisplatin, carboplatin, oxaliplatin, satraplatin, picoplatin, nedaplatin, triplatin, and phosphaplatins, such as those disclosed in U.S. Patent No. 8,034,964 and which are incorporated herein by reference. These compounds can be prepared by methods known in the art. Preferably, the anti-neoplastic agent is cisplatin. Cisplatin is a divalent inorganic water-soluble, platinum containing complex widely used to treat testicular, bladder, and ovarian cancers.

The platinum-based anti-neoplastic agents used in the therapeutic combinations of this invention can be combined with any pharmaceutically acceptable excipient, carrier, adjunct, or diluent known in the art.

The extract used in the therapeutic combinations of this invention can be derived from any species of the genus *Nerium.* Non-limiting examples include extracts from the species *Nerium indicum, Nerium oleander,* and *Nerium odorum.* Preferably, the extract is derived from *Nerium oleander,*

Extracts of *Nerium oleander* can be prepared by methods known in the art. Such methods include extraction with hot water (U.S. Patent Nos. 6,565,897 and 5,135,745), room temperature water and aqueous alcohol, particularly aqueous methanol and ethanol (U.S. Patent App. Pub. No. 2007/0154573 A1), supercritical carbon dioxide (U.S. Patent No. 7,402,325), and aloe (U.S. Patent App. Pub. No. 2010/0092585 A1). These aforementioned extraction methods disclosed in U.S. Patent Nos. 6,565,897, 5,135,745, and 7,402,325 and U.S. Patent App. Pub. Nos. 2007/0154573 A1 and 2010/0092585 A1 are incorporated herein by reference. Anvirzel™ is a hot water extract of *Nerium oleander* that contains oleandrin and oleandrigenin as its main constituents.

The extracts used in the combinations of the invention can contain any pharmaceutically acceptable excipient, carrier, adjunct, or diluent known in the art. Examples include mannitol, ascorbic acid, sodium ascorbate, methyl paraben, propyl paraben, and mixtures thereof.

Preferably, the therapeutic combination of the invention comprises cisplatin and an extract from the species *Nerium oleander.*

The anti-neoplastic agent comprising platinum and the extract from a species of the genus *Nerium* can be present in the therapeutic combinations of the invention as either separate or combined formulations.

The method of this invention for treating cancer comprises administering to a patient in need thereof a therapeutic combination comprising an anti-neoplastic agent comprising platinum and an extract from a species of the genus *Nerium.* As used herein, the term "patient" refers to a mammal afflicted with cancer. The preferred patient is a human.

The anti-neoplastic agent comprising platinum can be present in the therapeutic combinations of the invention and used in the method of this invention in an amount that, in the absence of the extract from a species of the genus *Nerium,* is either effective or ineffective to produce an increase in anti-neoplastic activity. An amount or dose that is "effective" is that which produces a particular cancer cell growth- or proliferation-inhibiting effect, tumor growth inhibiting effect, tumor volume increase-inhibiting effect, or cancer treatment effect in a cancer cell or tumor.

The anti-neoplastic agent comprising platinum can be administered to the patient via any mode known in the art, such as intravenously in the case of cisplatin, carboplatin, and oxalplatin, and orally in the case of satraplatin. A typical dosage of cisplatin for an adult is 70-100 mg/m² a day for three days, followed by two weeks off, constituting one cycle. Patients can receive a maximum of six cycles of therapy, but owing to toxicity problems most patients can only endure four cycles of such therapy. A typical adult (170 cm tall and weighing 70 kg), has 1.83 m² (The Journal of Pediatrics 1978 93:1:62-66; Gehan EA, George SL), corresponding to a dosage of about 125-185 mg.

The extract used in this method can be administered intramuscularly, sublingually, or intramuscularly and sublingually, either in a single dose or in multiple doses. The normal dosage for Anvirzel™ is 0.5 to 1.0 mL intramuscularly, 1.0 to 2.0 mL sublingually, or a combination of intramuscular and sublingual administration so that the total amount in a 24 hour period is no greater that 2 mL. Anvirzel™ administered sublingually only is divided into a series of smaller sub-doses, typically two to four doses of 0.5 mL or less per 24 hours.

As is common with pharmaceutical agents, the respective therapeutic doses of the components of the therapeutic combinations of the invention may vary with the condition of the patient and the route by which the drug is administered. The dose, and perhaps the dose frequency, will also vary according to the age, body weight, and response of the individual patient. It may be necessary to use dosages outside these ranges in some cases, as will be apparent to those skilled in the art. Further, it is noted that the clinician or treating physician will know how and when to interrupt, adjust, or terminate therapy in conjunction with individual patient response. The terms "therapeutic amount" and "therapeutically effective amount" are encompassed by the above-described dosage amounts and dose frequency schedules,

The therapeutic combinations of the invention can be administered to the patient simultaneously, separately, or sequentially.

The therapeutic combinations of the invention can be used according to this method to treat any type of cancer. Examples include prostate cancer, melanoma, pancreatic cancer, lung cancer, breast cancer, and colorectal cancer.

The following non-limiting examples demonstrate the synergistic cytostatic effect of coadministration of a *Nerium oleander* extract with cisplatin.

While these examples show certain specific embodiments of the invention, it will be manifest to those skilled in the art that various modifications and rearrangements of the parts may be made without departing from the spirit and scope of the underlying inventive concept and that the same is not limited to the particular forms herein shown and described.

### EXAMPLES

### Viability Assays

Three different viability assays were chosen to complement each other and yielded results dependent on concentration and cell line: the Methyl Tetrazolium dye assay, the Sulforhodamine B fluorescent dye assay, and the Crystal Violet dye assay.

The Methyl-Tetrazolium dye assay measures the activity of enzymes in mitochondria. The dye precursor is taken up by cells, where it undergoes reduction to the purple formazan. It measures only the mitochondrial activity and, thus, it is not always a reliable indication of whether the cells themselves are alive or dead, since even a recently dead cell has lower enzyme activity in mitochondria. Intracellular glucose concentration, pH, and time before assay all affect the measurements.

The Sulforhodamine B and Crystal Violet assays provide information about protein and were used to complement the Methyl-Tetrazolium dye assay. The Sulforhodamine B assay is more sensitive for the detection of small number of cells and shows a linear relationship between cell number and its staining intensity. It is used for the quantification of cellular proteins of cultured cells. The assay using Crystal Violet, a dye that binds electrostatically to proteins and stains DNA, provides a reliable, simple assay for measuring viability of cells.

The Methyl Tetrazolium, Sulforhodamine B, and Crystal Violet assays were used to assess cell viabilities. The incubation times were 24 hours, 48 hours, and 72 hours. The *Nerium oleander* extract Anvirzel™ (a hot water extract of oleander obtained from Salud Integral, Honduras) was tested in concentrations ranging from 0.01 ng/mL to 10 ng/mL. Cisplatin (Sigma, P4394) was tested in concentrations ranging from 0.1 µg/mL to 100 µg/mL.

Cells were detached by trypsinization (Trypsin-0.25% EDTA, Invitrogen, 25200-072) during the logarithmic phase of culture growth and plated in 96-well plates (18.000 cells/well) (Corning, Costar 359) in a final volume of 200µl of medium per well. After 70 to 80% confluence of the culture, the medium was removed and the Anvirzel™ diluted in water and cisplatin diluted in N,N-dimethylformamide (Fluka, 40255) were added to the cells in graduated densities. The absorbance was measured after 24 hours, 48 hours, and 72 hours of incubation.

For the Sulforhodamine B assay, 96-well plates were fixed by 10% trichloroacetic acid (Fluka, 91228) and were incubated at 4 °C for 1hour. Afterwards, plates were rinsed with water and cells were stained with 0.4% Sulforhodamine B (Sigma, 341738), dissolved in 1% acetic acid (Carlo Ebra, 401422) for 15 minutes at room temperature (RT). The unbound stain was washed twice with 1% acetic acid. Finally, 10 mM Tris Buffer pH 10.5 (Sigma, T6791) was added to dissolve the dye.

For the Crystal Violet assay, the medium was removed from the 96-well plates, the plates rinsed with PBS (Sigma, P3813) and then the cells were rinsed by the addition of 10% formalin (MERCK, 1.04003.2500) for 20 minutes at RT. Formalin was removed and 0.25% aqueous crystal violet (Sigma, HT901), dissolved in water, was added for 10 minutes at RT. Unbound Crystal Violet was rinsed by washing with water and finally 33% acetic acid was added to dissolve the dye.

For the Methyl-Tetrazolium assay, the dye (Sigma, M2128; 5 mg/mL, diluted in phosphate-buffered saline) was added to each well and plates were incubated for 3 hours at 37°C. After the end of the incubation period, the medium was discarded and the cells were rinsed with phosphate-buffered saline. Finally, the formazan crystals were dissolved in dimethylsulphoxide (Sigma, D4540).

The optical density of each plate was measured on a µQuant spectrophotometer and the data were analyzed with Gen5 software (µQuant Biomolecular Spectrophotometer MQX200 and Gen5™ Microplate Data Collection & Analysis software, BioTek® Instruments.Inc, April 2008). Absorbance was measured at 570 nm for all assays and a second wavelength was measured in order to subtract noise. For the Methyl-Tetrazolium assay this second wavelength was 630 nm and for the Sulforhodamine B and Crystal Violet assays it was 690 nm.

### Cell Lines

Human carcinoma cell lines were obtained by the European Collection of Cell Cultures from Health Protective Agency, UK, and included lines derived from human prostate cancer (PC3, LNCaP and 22Rv1), human breast cancer (MDA-MB 231, T47D, MCF-7), non-small cell lung carcinoma (CALU-1, COLO699N, COR-L 105), colorectal cancer (HCT-116, HT55, HCT-15), melanoma (A375), and pancreatic cancer (PANC-1).

Cells were cultured in 75 cm² flasks (Orange Scientific, 5520200, Belgium) in the medium indicated for each line with the appropriate amount of heat inactivated Fetal Bovine Serum (FBS, Invitrogen, 10106-169, California) and 2 mM L-Glutamine (Sigma, G5792, Germany) for each cell line and incubated at 37°C in a 5% CO₂ atmosphere.

### Statistical Analysis

All treatments for each cell line were performed in triplicate. The statistical significance of all effects was evaluated by "difference of the means" test (p < 0.05).

Anvirzel™ concentrations ranged from 0.01 ng/mL to 10 ng/mL, while those for cisplatin ranged from 0.1 µg/mL to 100 µg/mL. Surprisingly, lower concentrations of both the Anvirzel™ (0.01 to 0.1 ng/mL) and the cisplatin (0.1 µg/mL) gave better results than higher concentrations, which yielded unreliable and irreproducible results. The results were also time-dependent, as was observed after 48 and 72 hours of incubation.

### Example 1: LNCaP cell line

Table 1 below shows results for LNCaP cells, a prostatic cancer-derived cell line, with cell population densities estimated from optical absorbance data.

By Crystal Violet assay, after 48 hours of incubation, unstimulated cells showed an apparent absorbance of 1.307 (a dimensionless quantity), with essentially the same value (1.249) obtained in the presence of 0.0 1 ng/mL of Anvirzel™. The presence of 0.1µg/mL of cisplatin reduced the apparent absorbance to 0.408, but the presence of 0.01 of ng/mL Anvirzel™ and 0.1 µg/mL of cisplatin further reduced the value to 0.178 (p=0.00008<0.05). A similar diminution in apparent absorbance was found after 72 hours incubation as well.

By Sulforhodamine B assay, after 48 hours of incubation, unstimulated cells showed an apparent absorbance of 2.616, with essentially the same value (2.667) obtained in the presence of 0.01 ng/mL of Anvirzel™. The presence of 0.1 µg/mL of cisplatin reduced the apparent absorbance to 1.681, but the presence of 0.01 ng/mL of Anvirzel™ and 0.1 µg/mL of cisplatin further reduced the value to 1.033 (p=0.007<0.05). A similar diminution in apparent absorbance was found after 72 hours incubation as well.

By Methyl-Tetrazolium assay, after 48 hours of incubation, unstimulated cells showed an apparent absorbance of 0.429, with a somewhat lower value (0.292) obtained in the presence of 0.01 ng/mL of Anvirzel™. The presence of 0. 1 µg/mL of cisplatin reduced the apparent absorbance to 0.161, but the presence of 0.01 ng/mL of Anvirzel™ and 0.1 µg/mL of cisplatin further reduced the value to 0.033 (p=0.005<0.05). A similar diminution in apparent absorbance was found after 72 hours incubation as well.

**Table 1: Statistical evaluation of absorbance in LNCaP prostate cancer cell line.**

| **LNCaP Cell Line ABSORBANCE** | | |
|---|---|---|
| **Crystal Violet Assay** | **48h** | **72h** |
| Unstimulated cells | 1.307 | 2.08 |
| 0.01 ng/mL Anvirzel | 1.249 | 2.416 |
| 0.1µg/mL cisplatin | 0.408 | 0.448 |
| 0.01 ng/mL Anvirzel + 0.1µg/mL cisplatin | 0.178 (p=0.00008<0.05) | 0.187 (p=0.002<0.05) |

| **Sulforhodamine B Assay** | **48h** | **72h** |
|---|---|---|
| Unstimulated cells | 2.616 | 2.906 |
| 0. 0 1 ng/mL Anvirzel | 2.667 | 2.927 |
| 0.1 µg/mL cisplatin | 1.681 | 1.766 |
| 0.01 ng/mL Anvirzel + µg/mL cisplatin | 1.033 (p=0.007<0.05) | 1.095 (p=0.001<0.05) |

| **Methyl-Tetrazolium Assay** | **48h** | **72h** |
|---|---|---|
| Unstimulated cells | 0.429 | 0.553 |
| 0.01 ng/mL Anvirzel | 0.292 | 0.373 |
| 0.1 µg/mL cisplatin | 0.161 | 0.17 |
| 0.01 ng/mL Anvirzel + 0.1 µg/mL cisplatin | 0.033 (p=0.005<0.05) | 0.035 (p=0.0001<0.05) |

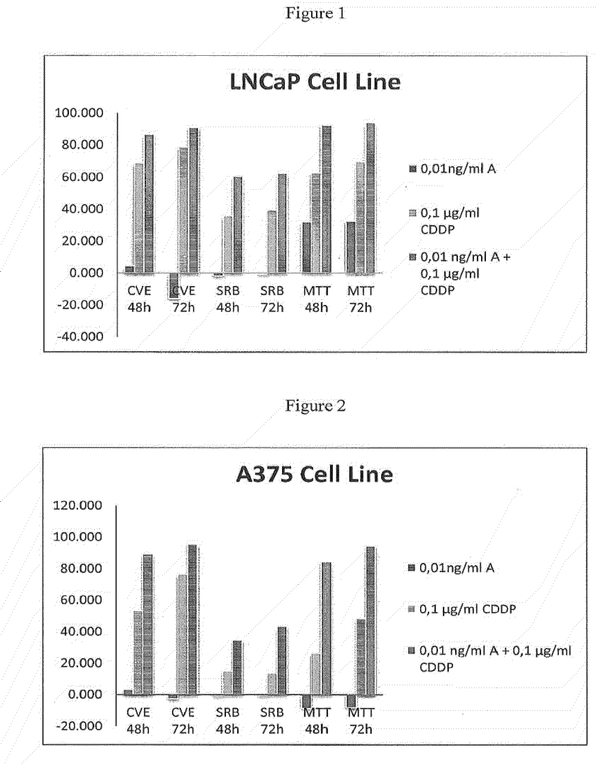

These results in Table 1 are graphically depicted in Figure 1.

### Example 2: A375 Cell Line

Table 2 below shows results for A375 cells, a melanoma-derived cell line, with cell population densities estimated from optical absorbance data,

By Crystal Violet assay, after 48 hours of incubation, unstimulated cells showed an apparent absorbance of 2.791, with essentially the same value (2.71) obtained in the presence of 0.01 ng/mL of Anvirzel™. The presence of 0.1 µg/mL of cisplatin reduced the apparent absorbance to 1.304, but the presence of 0.01 ng/mL of Anvirzel™ and 0.1 µg/mL of cisplatin reduced the value to 0.178 (p=0.00008<0.05). A similar diminution in apparent absorbance was found after 72 hours incubation as well.

By Sulforhodamine B assay, after 48 hours of incubation, unstimulated cells showed an apparent absorbance of 2.836, with essentially the same value (2.854) obtained in the presence of 0.01 ng/mL of Anvirzel™. The presence of 0.1 µg/mL of cisplatin reduced the apparent absorbance to 2.421, but the presence of 0. 0 1 ng/mL of Anvirzel™ of and 0.1 µg/mL of cisplatin further reduced the value to 1.89 (p=0.01<0.05). A similar diminution in apparent absorbance was found after 72 hours incubation as well.

By Methyl-Tetrazolium assay, after 48 hours of incubation, unstimulated cells showed an apparent absorbance of 0.45, with essentially the same value (0.49) obtained in the presence of 0.01 ng/mL of Anvirzel™. The presence of 0.1 µg/mL of cisplatin reduced the apparent absorbance to 0.332, but the presence of 0.01 ng/mL of Anvirzel™ and 0.1 µg/mL of cisplatin reduced the value to 0.072 (p=0.002<0.05). A similar diminution in apparent absorbance was found after 72 hours incubation as well.

**Table 2: Statistical evaluation of absorbance in A375 melanoma cancer cell line.**

| **A375 Cell Line ABSORBANCE** | | |
|---|---|---|
| **Crystal Violet Assay** | **48h** | **72h** |
| Unstimulated cells | 2.791 | 2.767 |
| 0.01 ng/mL Anvirzel | 2.71 | 2.846 |
| 0.1 µg/mL cisplatin | 1.304 | 0.663 |
| 0.01 ng/mL Anvirzel + 0.1 µg/mL cisplatin | 0.306 (p=0.002<0.05) | 0.123 (p=0.0009<0.05) |

| **Sulforhodamine B Assay** | **48h** | **72h** |
|---|---|---|
| Unstimulated cells | 2.836 | 2.867 |
| 0.01 ng/mL Anvirzel | 2.854 | 2.884 |
| 0.1 µg/mL cisplatin | 2.421 | 2.487 |
| 0.01 ng/mL Anvirzel + 0.1 µg/mL cisplatin | 1.89 (p=0.01<0.05) | 1.624 (p=0.006<0.05) |

| **Methyl-Tetrazolium Assay** | **48h** | **72h** |
|---|---|---|
| Unstimulated cells | 0.45 | 0.922 |
| 0.01 ng/mL Anvirzel | 0.49 | 0.999 |
| 0.1 µg/mL cisplatin | 0.332 | 0.479 |
| 0.01 ng/mL Anvirzel + 0.1 µg/mL cisplatin | 0.072 (p=0.002<0.05) | 0.053 (p=0.005<0.05) |

These results in Table 2 are graphically depicted in Figure 2.

### Example 3: PANC-1 Cell Line

Table 3 below shows results for PANC-1 cells, a pancreatic cancer-derived cell line, with cell population densities estimated from optical absorbance data.

By Crystal Violet assay, after 48 hours of incubation, unstimulated cells showed an apparent absorbance of 0.964, with essentially the same value (1.04) obtained in the presence of 0.01 ng/mL of Anvirzel™. The presence of 0.1 µg/mL of cisplatin reduced the apparent absorbance to 0.753, but the presence of 0.01 ng/mL of Anvirzel™ and 0.1 µg/mL of cisplatin reduced the value to 0.283 (p=0.01<0.05). A similar diminution in apparent absorbance was found after 72 hours incubation as well.

By Sulforhodamine B assay, after 48 hours of incubation, unstimulated cells showed an apparent absorbance of 2.332, with essentially the same value (2.328) obtained in the presence of 0.01 ng/mL of Anvirzel™. The presence of 0. 1 µg/mL of cisplatin reduced the apparent absorbance to 1.861, but the presence of 0.01 ng/mL of Anvirzel™ and 0.1 µg/mL of cisplatin further reduced the value to 1.25 (p=0.002<0.05). A similar diminution in apparent absorbance was found after 72 hours incubation as well.

By Methyl-Tetrazolium assay, after 48 hours of incubation, unstimulated cells showed an apparent absorbance of 0.546, with a similar value (0.518) obtained in the presence of 0.01 ng/mL of Anvirzel™. The presence of 0.1 µg/mL of cisplatin reduced the apparent absorbance to 0.286, but the presence of 0.01 ng/mL of Anvirzel™ and 0.1 µg/mL of cisplatin reduced the value to 0.143 (p=0.0007<0.05). A similar diminution in apparent absorbance was found after 72 hours incubation as well.

**Table 3: Statistical evaluation of absorbance in PANC-1 pancreatic cancer cell line.**

| **PANC-1 Cell Line ABSORBANCE** | | |
|---|---|---|
| **Crystal Violet Assay** | **48h** | **72h** |
| Unstimulated cells | 0.964 | 2.862 |
| 0.01 ng/mL Anvirzel | 1.04 | 2.711 |
| 0.1µg/mL cisplatin | 0.753 | 0.872 |
| 0.01 ng/mL Anvirzel + 0.1 µg/mL cisplatin | 0.283 (p=0.0 1<0.05) | 0.336 (p=0.001<0.05) |

| **Sulforhodamine B Assay** | **48h** | **72h** |
|---|---|---|
| Unstimulated cells | 2.332 | 2.924 |
| 0.01 ng/mL Anvirzel | 2.328 | 2.882 |
| 0.1 µg/mL cisplatin | 1.861 | 2.536 |
| 0.01 ng/mL Anvirzel + 0.1µg/mL cisplatin | 1.25 (p=0.002<0.05) | 1.837 (p=0.004<0.05) |

| **Methyl-Tetrazolium Assay** | **48h** | **72h** |
|---|---|---|
| Unstimulated cells | 0.546 | 1.012 |
| 0.01 ng/mL Anvirzel | 0.518 | 0.954 |
| 0.1µg/mL cisplatin | 0.286 | 0.344 |
| 0.01 ng/mL Anvirzel + 0.1µg/mL cisplatin | 0.143 (p=0.0007<0.05) | 0.115 (p=0.002<0.05) |

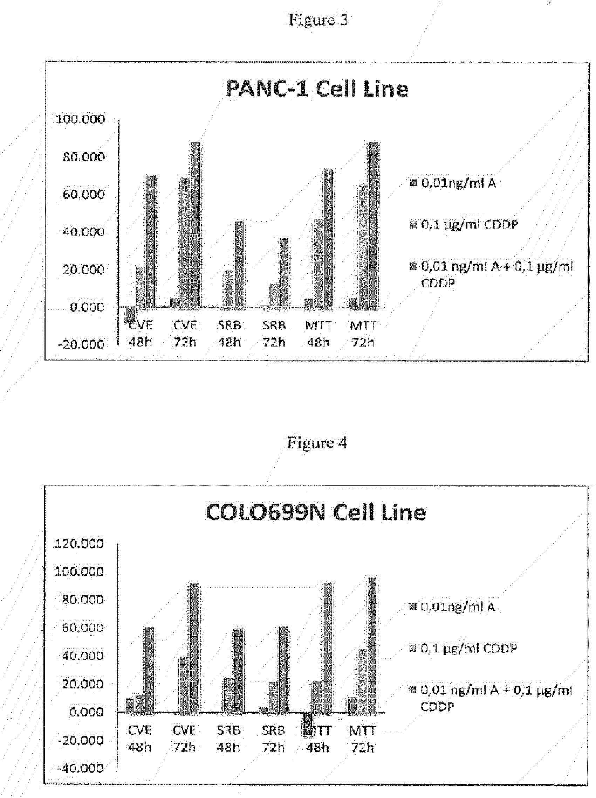

These results in Table 3 are graphically depicted in Figure 3.

### Example 4: COLO699N Cell Line

Table 4 below shows results for COLO699N cells, a lung cancer-derived cell line, with cell population densities estimated from optical absorbance data.

By Crystal Violet assay, after 48 hours of incubation, unstimulated cells showed an apparent absorbance of 0.918, with a similar value (0.826) obtained in the presence of 0.01 ng/mL of Anvirzel™. The presence of 0.1 µg/mL of cisplatin reduced the apparent absorbance slightly to 0.801, but the presence of 0.01 ng/mL of Anvirzel™ and 0.1 µg/mL of cisplatin reduced the value to 0.361 (p=0.01<0.05). A similar diminution in apparent absorbance was found after 72 hours incubation as well.

By Sulforhodamine B assay, after 48 hours of incubation, unstimulated cells showed an apparent absorbance of 2.636, with essentially the same value (2.633) obtained in the presence of 0.01 ng/mL of Anvirzel™. The presence of 0.1 µg/mL of cisplatin reduced the apparent absorbance to 1.975, but the presence of 0.01 ng/mL of Anvirzel™ and 0.1 µg/mL of cisplatin further reduced the value to 1.046 (p=0.00001<0.05). A similar diminution in apparent absorbance was found after 72 hours incubation as well.

By Methyl-Tetrazolium assay, after 48 hours of incubation, unstimulated cells showed an apparent absorbance of 0.82, with a somewhat higher value (0.953) obtained in the presence of 0.01 ng/mL of Anvirzel™. The presence of 0.1 µg/mL of cisplatin reduced the apparent absorbance to 0.636, but the presence of 0.01 ng/mL of Anvirzel™ and 0.1 µg/mL of cisplatin reduced the value to 0.058 (p=0.001 <0.05). A similar diminution in apparent absorbance was found after 72 hours incubation as well.

**Table 4: Statistical evaluation of absorbance in COLO699N lung cancer cell line.**

| **COLO699N Cell Line ABSORBANCE** | | |
|---|---|---|
| **Crystal Violet Assay** | **48h** | **72h** |
| Unstimulated cells | 0.918 | 1.659 |
| 0.01 ng/mL Anvirzel | 0.826 | 1.654 |
| 0.1µg/mL cisplatin | 0.801 | 1 |
| 0.01 ng/mL Anvirzel + 0.1 µg/mL cisplatin | 0.361 (p=0.01<0.05) | 0.138 (p=0.005<0.05) |

| **Sulforhodamine B Assay** | **48h** | **72h** |
|---|---|---|
| Unstimulated cells | 2.636 | 2.758 |
| 0.01 ng/mL Anvirzel | 2.633 | 2.655 |
| 0.1µg/mL cisplatin | 1.975 | 2.145 |
| 0.01 ng/mL Anvirzel + 0.1 µg/mL cisplatin | 1.046 (p=0.00001<0.05) | 1.062 (p=0.00009<0.05) |

| **Methyl-Tetrazolium Assay** | **48h** | **72h** |
|---|---|---|
| Unstimulated cells | 0.82 | 1.34 |
| 0.01 ng/mL Anvirzel | 0.953 | 1.184 |
| 0.1 µg/mL cisplatin | 0.636 | 0.73 |
| 0.01 ng/mL Anvirzel + 0.1µg/mL cisplatin | 0.058 (p=0.001<0.05) | 0.047 (p=0.001<0.05) |

These results in Table 4 are graphically depicted in Figure 4.

The following numbered paragraphs set out preferred features and preferred combinations of features of the present invention.
1. A therapeutic combination comprising (I) an anti-neoplastic agent comprising platinum and (II) an extract from a species of the genus *Nerium.*
2. The therapeutic combination of para 1, wherein (I) is present in an amount that, in the absence of (II), is effective to produce an increase in anti-neoplastic activity.
3. The therapeutic combination of para 1, wherein (I) is present in an amount that, in the absence of (II), is ineffective to produce an increase in anti-neoplastic activity.
4. The therapeutic combination of para 1, wherein (I) is selected from the group consisting of cisplatin, carboplatin, oxaliplatin, satraplatin, picoplatin, nedaplatin, triplatin, and phosphaplatins.
5. The therapeutic combination of para 1, wherein (I) is cisplatin.
6. The therapeutic combination of para 1, wherein (II) is an extract from a species selected from the group consisting of *Nerium indicum, Nerium oleander,* and *Nerium odorum.*
7. The therapeutic combination of para 1, wherein (II) is an extract from the species *Nerium oleander.*
8. The therapeutic combination of para 1, wherein (II) is a water-based extract.
9. The therapeutic combination of para 1, wherein (II) is an aloe-based extract.
10. The therapeutic combination of para 1, wherein (I) is cisplatin and (II) is an extract from the species *Nerium oleander.*
11. The therapeutic combination of para 1, wherein (I) and (II) are present as separate formulations.
12. A method for treating cancer comprising administering to a patient in need thereof a therapeutically effective amount of the therapeutic combination of para 1.
13. The method of para 12, wherein (I) is present in an amount that, in the absence of (II), is effective to produce an increase in anti-neoplastic activity.
14. The method of para 12, wherein (I) is present in an amount that, in the absence of (II), is ineffective to produce an increase in anti-neoplastic activity.
15. The method of para 12, wherein (I) is selected from the group consisting of cisplatin, carboplatin, oxaliplatin, satraplatin, picoplatin, nedaplatin, triplatin, and phosphaplatins.
16. The method of para 12, wherein (I) is cisplatin.
17. The method of para 12, wherein (II) is an extract from a species selected from the group consisting of *Nerium indicum*, *Nerium oleander*, and *Nerium odorum.*
18. The method of para 12, wherein (II) is an extract from the species *Nerium oleander.*
19. The method of para 12, wherein (II) is a water-based extract.
20. The method of para 12, wherein (II) is an aloe-based extract.
21. The method of para 12, wherein (I) is cisplatin and (II) is an extract from the species *Nerium oleander.*
22. The method of para 12, wherein (I) and (II) of said therapeutic combination are administered to the patient simultaneously, separately, or sequentially.
23. The method of para 12, wherein (II) is administered to the patient intramuscularly, sublingually, or intramuscularly and sublingually.
24. The method of para 12, wherein (II) is administered to the patient sublingually in two or more doses.
25. The method of para 12, wherein the cancer treated is prostate cancer, melanoma, pancreatic cancer, lung cancer, breast cancer, or colorectal cancer.

## Claims

1. A therapeutic combination comprising (I) an anti-neoplastic agent comprising platinum and (II) a hot water-based extract from a species of the genus *Nerium.*

2. The therapeutic combination of claim 1, wherein (I) is present in an amount that, in the absence of (II), is effective to produce an increase in anti-neoplastic activity.

3. The therapeutic combination of claim 1, wherein (I) is present in an amount that, in the absence of (II), is ineffective to produce an increase in anti-neoplastic activity.

4. The therapeutic combination of claim 1, wherein (I) is selected from the group consisting of cisplatin, carboplatin, oxaliplatin, satraplatin, picoplatin, nedaplatin, triplatin, and phosphaplatins.

5. The therapeutic combination of claim 1, wherein (I) is cisplatin.

6. The therapeutic combination of claim 1, wherein (II) is an extract from a species selected from the group consisting of *Nerium indicum*, *Nerium oleander*, and *Nerium odorum.*

7. The therapeutic combination of claim 1, wherein (II) is an extract from the species *Nerium oleander.*

8. The therapeutic combination of claim 1, wherein (II) is a water-based extract.

9. The therapeutic combination of claim 1, wherein (II) is an aloe-based extract.

10. The therapeutic combination of claim 1, wherein (I) is cisplatin and (II) is an extract from the species *Nerium oleander.*

11. The therapeutic combination of claim 1, wherein (I) and (II) are present as separate formulations.

12. A therapeutic combination according to any one of claims 1 to 10 for use in treating cancer.

13. The therapeutic combination for use according to claim 12, wherein (I) and (II) of said therapeutic combination are administered to the patient simultaneously, separately, or sequentially.

14. The therapeutic combination for use according to claim 12, wherein:
a) (II) is administered to the patient intramuscularly, sublingually, or intramuscularly and sublingually; or
b) (II) is administered to the patient sublingually in two or more doses.

15. The therapeutic combination for use according to claim 12, wherein the cancer treated is prostate cancer, melanoma, pancreatic cancer, lung cancer, breast cancer, or colorectal cancer.
